# EUROPEAN PATENT SPECIFICATION

(11) **EP 2 922 466 B1**
(45) Date of publication and mention of the grant of the patent: **14.10.2020**
(21) Application number: 13856706.0
(22) Date of filing: 15.11.2013
(51) Int. Cl.: A61B 5/0476

(54) **METHOD AND SYSTEM FOR DISPLAYING THE AMOUNT OF ARTIFACT PRESENT IN AN EEG RECORDING**
VERFAHREN UND SYSTEM ZUR ANZEIGE DER MENGE VON ARTEFAKTEN IN EINER EEG-AUFZEICHNUNG
PROCÉDÉ ET SYSTÈME POUR AFFICHER LA QUANTITÉ D'ARTEFACT PRÉSENTE DANS UN ENREGISTREMENT D'EEG

(30) Priority: 26.11.2012 US 201261729775 P; 14.03.2013 US 201313802765
(43) Date of publication of application: 30.09.2015
(73) Proprietor: Persyst Development Corporation, Solana Beach, CA 92075 (US)
(72) Inventor: NIERENBERG, Nicolas, San Diego, CA 92130 (US); WILSON, Scott, B., San Diego, CA 92130 (US); SCHEUER, Mark, L., San Diego, CA 92130 (US)
(74) Representative: Tomkins & Co
(86) International application number: PCT/US2013/070221
(87) International publication number: WO 2014/081620

(56) References cited:
- EP-A1- 1 250 886
- EP-A2- 0 504 027
- WO-A1-2011/149752
- WO-A2-2007/144307
- RU-C1- 2 076 625
- US-A1- 2005 144 042
- US-A1- 2008 027 515
- US-A1- 2011 130 675

## Description

### Technical Field

The present invention generally relates to a method and system for displaying EEG data. More specifically, the present invention relates to displaying an amount of artifact present in an EEG recording.

### Background Art

An electroencephalogram ("EEG") is a diagnostic tool that measures and records the electrical activity of a person's brain in order to evaluate cerebral functions. Multiple electrodes are attached to a person's head and connected to a machine by wires. The machine amplifies the signals and records the electrical activity of a person's brain. The electrical activity is produced by the summation of neural activity across a plurality of neurons. These neurons generate small electric voltage fields. The aggregate of these electric voltage fields create an electrical reading which electrodes on the person's head are able to detect and record. An EEG is a superposition of multiple simpler signals. In a normal adult, the amplitude of an EEG signal typically ranges from 1 micro-Volt to 100 micro-Volts, and the EEG signal is approximately 10 to 20 milli-Volts when measured with subdural electrodes. The monitoring of the amplitude and temporal dynamics of the electrical signals provides information about the underlying neural activity and medical conditions of the person.

An EEG is performed to: diagnose epilepsy; verify problems with loss of consciousness or dementia; verify brain activity for a person in a coma; study sleep disorders, monitor brain activity during surgery, and additional physical problems.

Multiple electrodes (typically 17-21, however there are standard positions for at least 70) are attached to a person's head during an EEG. The electrodes are referenced by the position of the electrode in relation to a lobe or area of a person's brain. The references are as follows: F = frontal; Fp = frontopolar; T = temporal; C = central; P = parietal; O = occipital; and A = auricular (ear electrode). Numerals are used to further narrow the position and "z" points relate to electrode sites in the midline of a person's head. An electrocardiogram ("EKG") may also appear on an EEG display.

The EEG records brain waves from different amplifiers using various combinations of electrodes called montages. Montages are generally created to provide a clear picture of the spatial distribution of the EEG across the cortex. A montage is an electrical map obtained from a spatial array of recording electrodes and preferably refers to a particular combination of electrodes examined at a particular point in time.

In bipolar montages, consecutive pairs of electrodes are linked by connecting the electrode input 2 of one channel to input 1 of the subsequent channel, so that adjacent channels have one electrode in common. The bipolar chains of electrodes may be connected going from front to back (longitudinal) or from left to right (transverse). In a bipolar montage signals between two active electrode sites are compared resulting in the difference in activity recorded. Another type of montage is the referential montage or monopolar montage. In a referential montage, various electrodes are connected to input 1 of each amplifier and a reference electrode is connected to input 2 of each amplifier. In a reference montage, signals are collected at an active electrode site and compared to a common reference electrode.

Reference montages are good for determining the true amplitude and morphology of a waveform. For temporal electrodes, CZ is usually a good scalp reference.

Being able to locate the origin of electrical activity ("localization") is critical to being able to analyze the EEG. Localization of normal or abnormal brain waves in bipolar montages is usually accomplished by identifying "phase reversal," a deflection of the two channels within a chain pointing to opposite directions. In a referential montage, all channels may show deflections in the same direction. If the electrical activity at the active electrodes is positive when compared to the activity at the reference electrode, the deflection will be downward. Electrodes where the electrical activity is the same as at the reference electrode will not show any deflection. In general, the electrode with the largest upward deflection represents the maximum negative activity in a referential montage.

Some patterns indicate a tendency toward seizures in a person. A physician may refer to these waves as "epileptiform abnormalities" or "epilepsy waves." These include spikes, sharp waves, and spike-and-wave discharges. Spikes and sharp waves in a specific area of the brain, such as the left temporal lobe, indicate that partial seizures might possibly come from that area. Primary generalized epilepsy, on the other hand, is suggested by spike-and-wave discharges that are widely spread over both hemispheres of the brain, especially if they begin in both hemispheres at the same time.

There are several types of brain waves: alpha waves, beta waves, delta wave, theta waves and gamma waves. Alpha waves have a frequency of 8 to 12 Hertz ("Hz"). Alpha waves are normally found when a person is relaxed or in a waking state when a person's eyes are closed but the person is mentally alert. Alpha waves cease when a person's eyes are open or the person is concentrating. Beta waves have a frequency of 13Hz to 30Hz. Beta waves are normally found when a person is alert, thinking, agitated, or has taken high doses of certain medicines. Delta waves have a frequency of less than 3Hz. Delta waves are normally found only when a person is asleep (non-REM or dreamless sleep) or the person is a young child. Theta waves have a frequency of 4Hz to 7Hz. Theta waves are normally found only when the person is asleep (dream or REM sleep) or the person is a young child. Gamma waves have a frequency of 30Hz to 100Hz. Gamma waves are normally found during higher mental activity and motor functions.

The following definitions are used herein.

"Amplitude" refers to the vertical distance measured from the trough to the maximal peak (negative or positive). It expresses information about the size of the neuron population and its activation synchrony during the component generation.

The term "analogue to digital conversion" refers to when an analogue signal is converted into a digital signal which can then be stored in a computer for further processing. Analogue signals are "real world" signals (e.g., physiological signals such as electroencephalogram, electrocardiogram or electrooculogram). In order for them to be stored and manipulated by a computer, these signals must be converted into a discrete digital form the computer can understand.

"Artifacts" are electrical signals detected along the scalp by an EEG, but that originate from non-cerebral origin. There are patient related artifacts (e.g., movement, sweating, ECG, eye movements) and technical artifacts (50/60 Hz artifact, cable movements, electrode paste-related).

The term "differential amplifier" refers to the key to electrophysiological equipment. It magnifies the difference between two inputs (one amplifier per pair of electrodes).

"Duration" is the time interval from the beginning of the voltage change to its return to the baseline. It is also a measurement of the synchronous activation of neurons involved in the component generation.

"Electrode" refers to a conductor used to establish electrical contact with a nonmetallic part of a circuit. EEG electrodes are small metal discs usually made of stainless steel, tin, gold or silver covered with a silver chloride coating. They are placed on the scalp in special positions.

"Electrode gel" acts as a malleable extension of the electrode, so that the movement of the electrodes leads is less likely to produce artifacts. The gel maximizes skin contact and allows for a low-resistance recording through the skin.

The term "electrode positioning" (10/20 system) refers to the standardized placement of scalp electrodes for a classical EEG recording. The essence of this system is the distance in percentages of the 10/20 range between Nasion-Inion and fixed points. These points are marked as the Frontal pole (Fp), Central (C), Parietal (P), occipital (O), and Temporal (T). The midline electrodes are marked with a subscript z, which stands for zero. The odd numbers are used as subscript for points over the left hemisphere, and even numbers over the right

"Electroencephalogram" or "EEG" refers to the tracing of brain waves, by recording the electrical activity of the brain from the scalp, made by an electroencephalograph.

"Electroencephalograph" refers to an apparatus for detecting and recording brain waves (also called *encephalograph)*.

"Epileptiform" refers to resembling that of epilepsy.

"Filtering" refers to a process that removes unwanted frequencies from a signal.

"Filters" are devices that alter the frequency composition of the signal.

"Montage" means the placement of the electrodes. The EEG can be monitored with either a bipolar montage or a referential one. Bipolar means that there are two electrodes per one channel, so there is a reference electrode for each channel. The referential montage means that there is a common reference electrode for all the channels.

"Morphology" refers to the shape of the waveform. The shape of a wave or an EEG pattern is determined by the frequencies that combine to make up the waveform and by their phase and voltage relationships. Wave patterns can be described as being: "Monomorphic". Distinct EEG activity appearing to be composed of one dominant activity. "Polymorphic", distinct EEG activity composed of multiple frequencies that combine to form a complex waveform. "Sinusoidal". Waves resembling sine waves. Monomorphic activity usually is sinusoidal. "Transient". An isolated wave or pattern that is distinctly different from background activity.

"Spike" refers to a transient with a pointed peak and a duration from 20 to under 70 msec.

The term "sharp wave" refers to a transient with a pointed peak and duration of 70-200 msec.

The term "neural network algorithms" refers to algorithms that identify sharp transients that have a high probability of being epileptiform abnormalities.

"Noise" refers to any unwanted signal that modifies the desired signal. It can have multiple sources.

"Periodicity" refers to the distribution of patterns or elements in time (e.g., the appearance of a particular EEG activity at more or less regular intervals). The activity may be generalized, focal or lateralized.

An EEG epoch is an amplitude of a EEG signal as a function of time and frequency.

An EEG report produces tremendous amounts of information about a person's brain activity. However, there is a need to quickly and easily interpret that information in order to properly analyze the brain activity of a person. US 2011/0130675 A1 discloses a multi-channel brain or cortical activity monitoring system. EP 1 250 886 A1 discloses an anesthesia monitoring system based on EEG signals.

### Summary of the Invention

The present invention relates to a method and system for displaying an amount of artifact present in an EEG recording, as defined in the appended claims. While developing artifact reduction techniques, the inventors realized that displaying in graphical form the amount of artifact present in an EEG record would be very helpful to a reviewer of the EEG recording. Thus, the inventors invented a method and system to visually display in graphical form the amount of artifact present in an EEG record.

Displaying in graphical form the amount of artifact present in an EEG record allows a reviewer of the EEG recording to see how much muscle and eye movement is present in the EEG record.

Displaying in graphical form the amount of artifact present in an EEG record provides an un-complex indication of a patient state in summary form.

Displaying in graphical form the amount of artifact present in an EEG record indicates if there is artifact remaining in the trends.

The present invention is a graphical representation of the amount of artifact of various types present in an EEG record displayed over time.

A graphical representation of the amount of artifact of various types present in an EEG record is shown as a series of horizontal lines. One horizontal line each for muscle artifacts, chewing artifacts, vertical eye movement artifacts, and lateral eye movement artifacts. Optionally, the depth of color of the horizontal line indicates the amount of that artifact detected in a given time period.

One aspect of the present invention is a method for displaying an amount of artifact present in an EEG recording. The method includes generating an EEG recording from a machine comprising a plurality of electrodes, an amplifier and processor. The method also includes analyzing the EEG recording to determine an amount of artifact present in the EEG recording. The method also includes graphically displaying the amount of artifact present in an EEG recording on a display, wherein the amount of the plurality of artifact types present in the EEG recording is shown as a plurality of horizontal lines, wherein the plurality of horizontal lines comprises a horizontal line for a muscle artifact, a horizontal line for a chewing artifact, a horizontal line for a vertical eye movement artifact, and a horizontal line for a lateral eye movement artifact.

Another aspect of the present invention is a system for displaying an amount of artifact present in an EEG recording. The system includes electrodes, a processor, and a display. The electrodes generate a plurality of EEG signals. The processor is connected to the electrodes and the processor is configured to generate an EEG recording from the plurality of EEG signals. The display is connected to the processor and displays an EEG recording. The processor is configured to graphically display an amount of artifact present in an EEG recording, wherein the amount of the plurality of artifact types present in the EEG recording is shown as a plurality of horizontal lines, wherein the plurality of horizontal lines comprises a horizontal line for a muscle artifact, a horizontal line for a chewing artifact, a horizontal line for a vertical eye movement artifact, and a horizontal line for a lateral eye movement artifact.

### Brief Description of the Drawings

FIG. 1 is a graphical display of the amount of artifact present in an EEG recording.
FIG. 1A is a graphical display of the amount of artifact present in an EEG recording.
FIG. IB is an enlarged and isolated view of a box IB of a seizure probability channel of FIG. 1.
FIG. 1C is an enlarged and isolated view of horizontal lines of the artifact intensity channel of FIG. 1.
FIG. 2 is a block diagram of a system for analyzing an EEG recording.
FIG. 3 is a map for electrode placement for an EEG.
FIG. 4 is a detailed map for electrode placement for an EEG.
FIG. 5 is an illustration of a CZ reference montage.
FIG. 6 is an illustration of an EEG recording containing a seizure, a muscle artifact and an eye movement artifact.
FIG. 7 is an illustration of the EEG recording of FIG. 6 with the muscle artifact removed.
FIG. 8 is an illustration of the EEG recording of FIG. 7 with the eye movement artifact removed.

### Best Mode(s) For Carrying Out The Invention

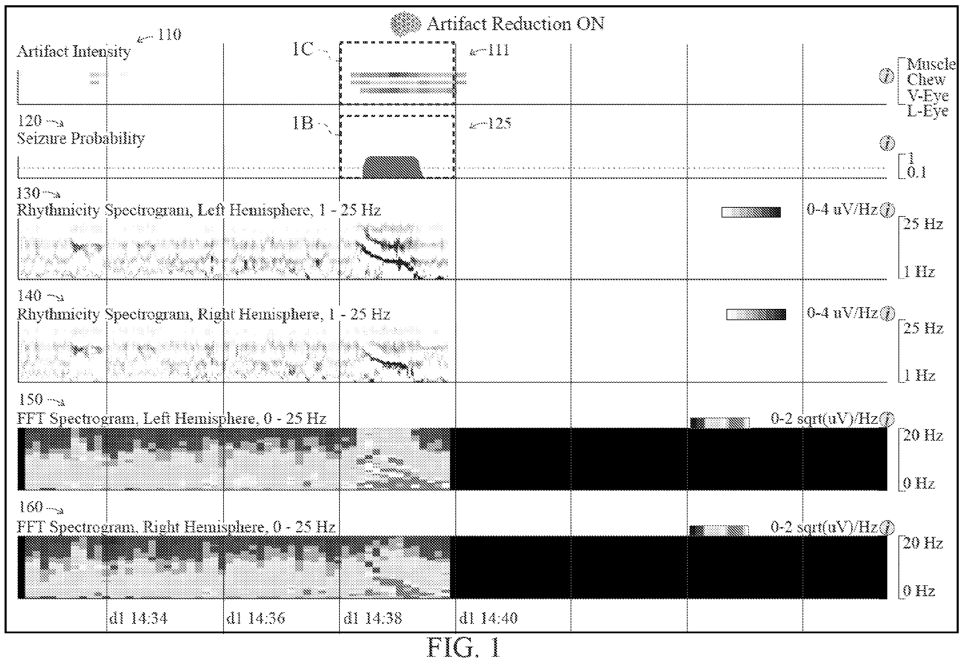

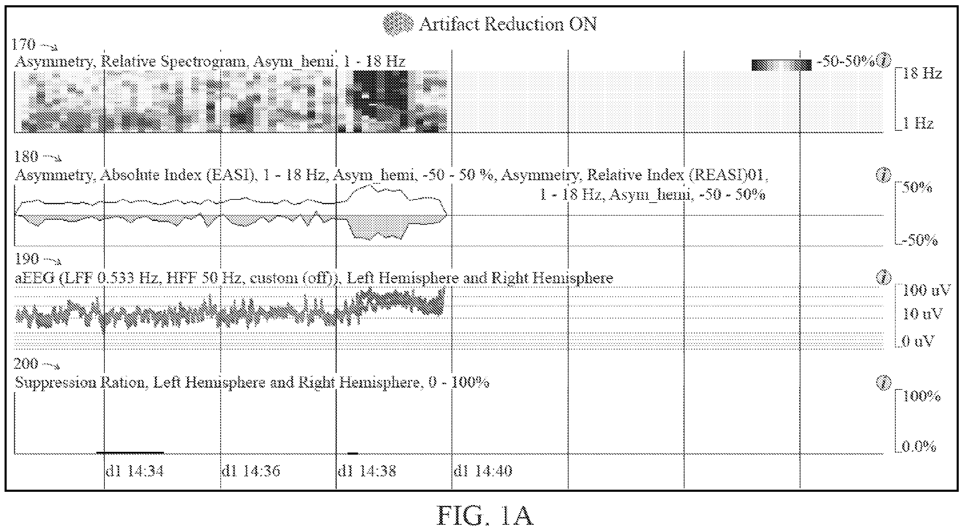

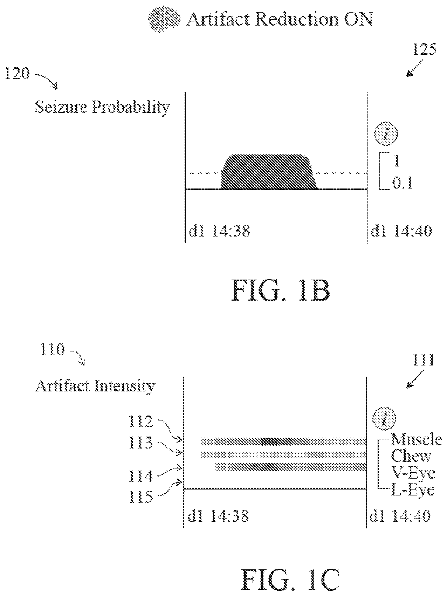

FIGS. 1, 1A, IB and 1C illustrate a graphical display of the amount of artifact present in an EEG recording. An artifact intensity channel 1 10 is shown as a series of horizontal lines 111. The plurality of horizontal lines 111 shown comprises a horizontal line 112 for a muscle artifact, a horizontal line 113 for a chewing artifact, a horizontal line 114 for a vertical eye movement artifact, and a horizontal line 1 15 for a lateral eye movement artifact. Those skilled in the pertinent art will recognize that more horizontal lines may be used without departing from the scope of the present invention.

Also shown in FIGS. 1 and 1A are a seizure probability channel 120, a rhythmicity spectrogram, left hemisphere channel 130, a rhythmicity spectrogram, right hemisphere channel 140, a FFT spectrogram left hemisphere channel 150, a FFT spectrogram right hemisphere channel 160, an asymmetry relative spectrogram channel 170, a asymmetry absolute index channel 180, an aEEG channel 190, and a suppression ration, left hemisphere and right hemisphere channel 200.

Rhythmicity spectrograms allow one to see the evolution of seizures in a single image. The rhythmicity spectrogram measures the amount of rhythmicity which is present at each frequency in an EEG record.

The seizure probability trend shows a calculated probability of seizure activity over time. The seizure probability trend shows the duration of detected seizures, and also suggests areas of the record that may fall below the seizure detection cutoff, but are still of interest for review. The seizure probability trend when displayed along with other trends, provides a comprehensive view of quantitative changes in an EEG.

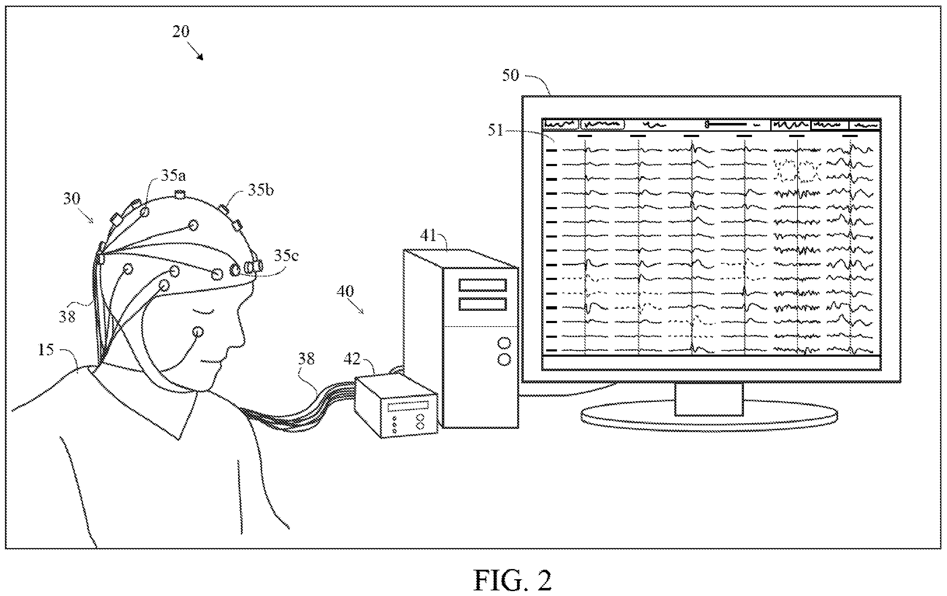

FIG. 2 illustrates a system 20 for a user interface for automated artifact filtering for an EEG. A patient 15 wears an electrode cap 31, consisting of a plurality of electrodes 35a-35c, attached to the patient's head with wires 38 from the electrodes 35 connected to an EEG machine component 40 which consists of an amplifier 42 for amplifying the signal to a computer 41 with a processor, which is used to analyze the signals from the electrodes 35 and create an EEG recording 51, which can be viewed on a display 50. A more thorough description of an electrode utilized with the present invention is detailed in Wilson et al, U.S. Patent Number 8112141 for a Method And *Device For Quick Press On EEG Electrode.* The EEG is optimized for automated artifact filtering. The EEG recordings are then processed using neural network algorithms to generate a processed EEG recording which is analyzed for display.

An additional description of analyzing EEG recordings is set forth in Wilson et al, U.S. Patent Application Number 13/620855, filed on September 15, 2012, for a *Method And System For Analyzing An EEG Recording.*

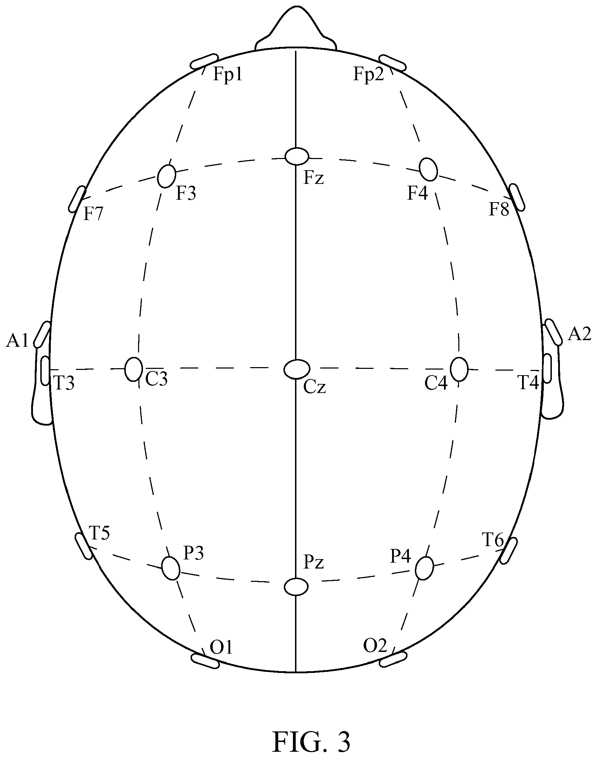

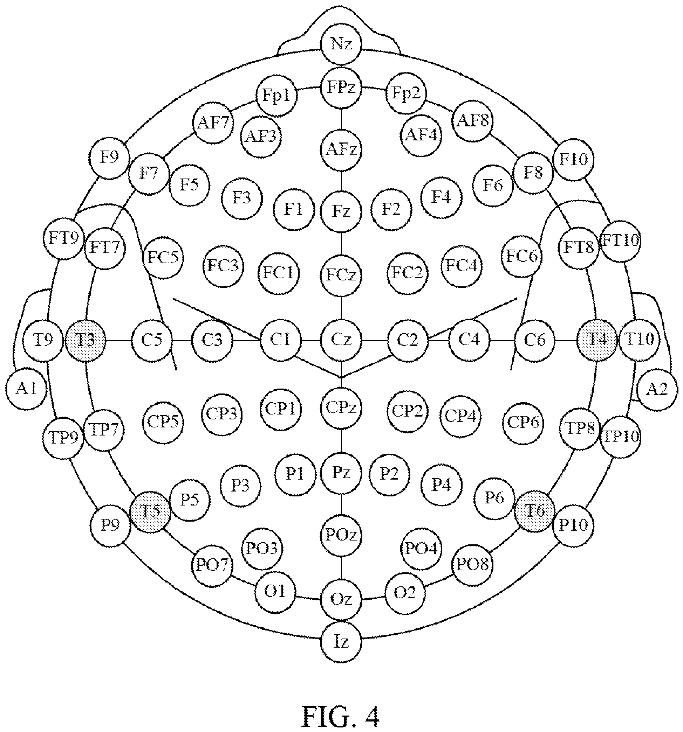

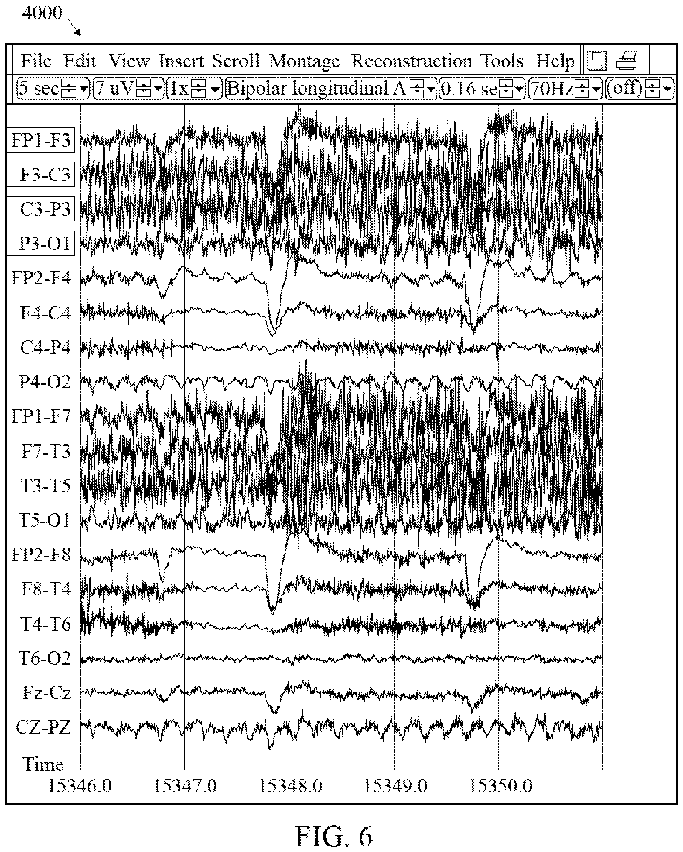

A patient has a plurality of electrodes attached to the patient's head with wires from the electrodes connected to an amplifier for amplifying the signal to a processor, which is used to analyze the signals from the electrodes and create an EEG recording. The brain produces different signals at different points on a patient's head. Multiple electrodes are positioned on a patient's head as shown in FIGS. 3 and 4. The CZ site is in the center. For example, Fpl on FIG. 4 is represented in channel FP1-F3 on FIG. 6. The number of electrodes determines the number of channels for an EEG. A greater number of channels produce a more detailed representation of a patient's brain activity. Preferably, each amplifier 42 of an EEG machine component 40 corresponds to two electrodes 35 attached to a head of the patient 15. The output from an EEG machine component 40 is the difference in electrical activity detected by the two electrodes. The placement of each electrode is critical for an EEG report since the closer the electrode pairs are to each other, the less difference in the brainwaves that are recorded by the EEG machine component 40.

The EEG is optimized for automated artifact filtering. The EEG recordings are then processed using neural network algorithms to generate a processed EEG recording, which is analyzed for display. During acquisition of the EEG recording, a processing engine performs continuous analysis of the EEG waveforms and determines the presence of most types of electrode artifact on a channel-by-channel basis. Much like a human reader, the processing engine detects artifact by analyzing multiple features of the EEG traces. The preferred artifact detection is independent of impedance checking. During acquisition the processing monitors the incoming channels looking for electrode artifacts. When artifacts are detected they are automatically removed from the seizure detection process and optionally removed from the trending display. This results in much a much higher level of seizure detection accuracy and easier to read trends than in previous generation products.

Algorithms for removing artifact from EEG typically use Blind Source Separation (BSS) algorithms like CCA (canonical correlation analysis) and ICA (Independent Component Analysis) to transform the signals from a set of channels into a set of component waves or "sources."

I In one example an algorithm called BSS-CCA is used to remove the effects of muscle activity from the EEG. Using the algorithm on the recorded montage will frequently not produce optimal results. In this case it is generally optimal to use a montage where the reference electrode is one of the vertex electrodes such as CZ in the international 10-20 standard. In this algorithm the recorded montage would first be transformed into a CZ reference montage prior to artifact removal. In the event that the signal at CZ indicates that it is not the best choice then the algorithm would go down a list of possible reference electrodes in order to find one that is suitable.

It is possible to perform BSS-CCA directly on the user-selected montage. However this has two issues. First this requires doing an expensive artifact removal process on each montage selected for viewing by the user. Second the artifact removal will vary from one montage to another, and will only be optimal when a user selects a referential montage using the optimal reference. Since a montage that is required for reviewing an EEG is frequently not the same as the one that is optimal for removing artifact this is not a good solution.

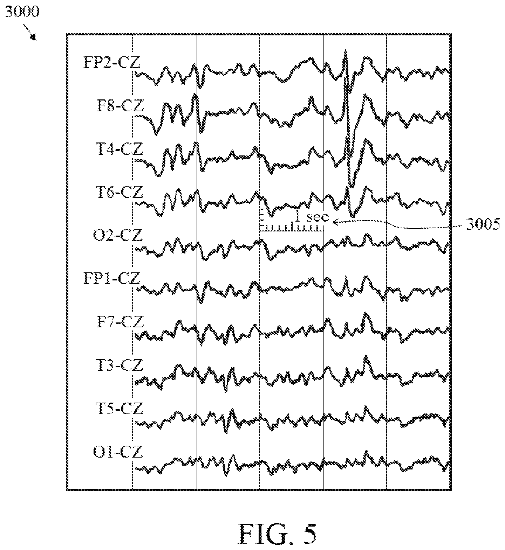

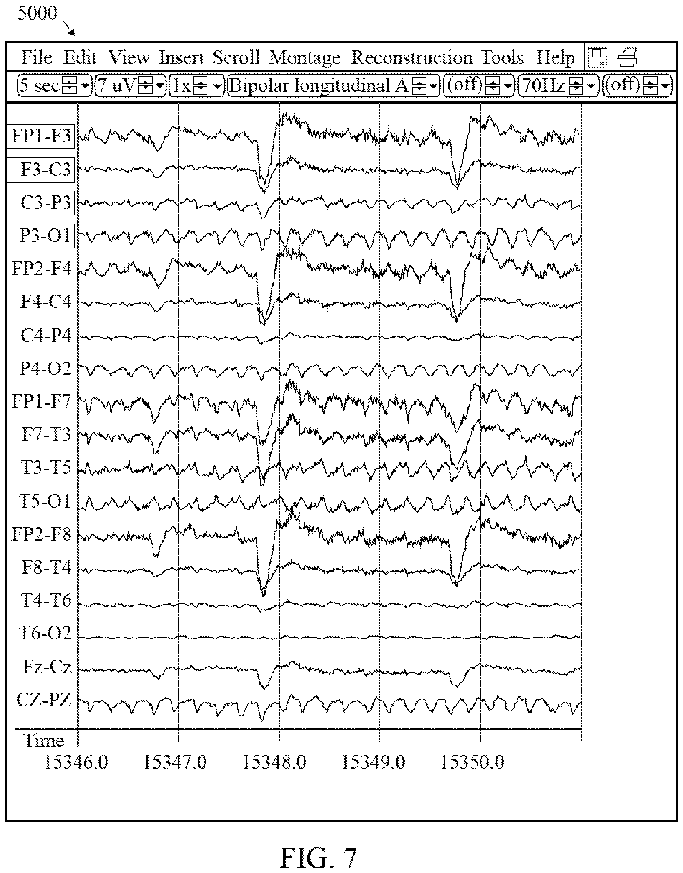

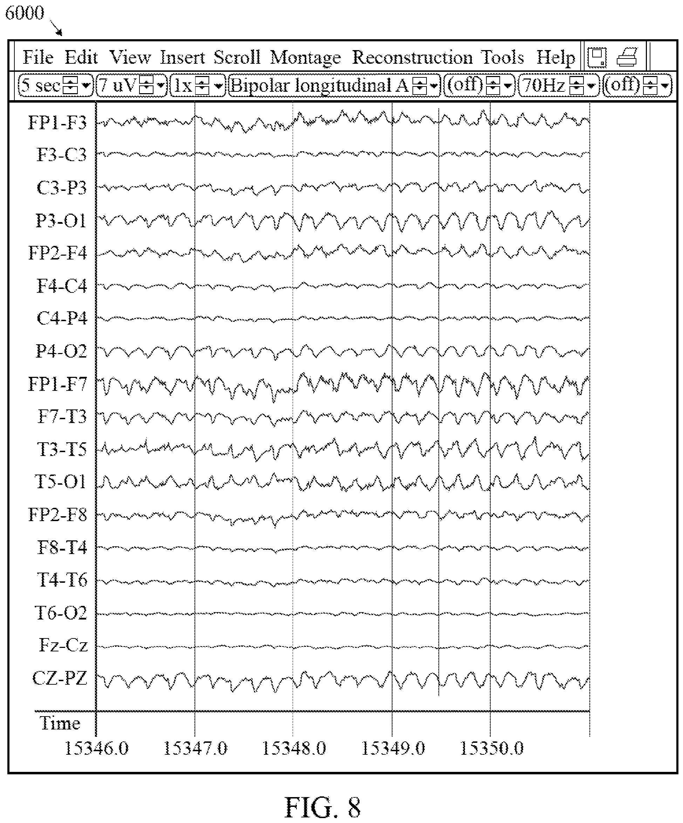

The FIGS. 5-8 illustrate how removing artifacts from the EEG signal allow for a clearer illustration of a brain's true activity for the reader. FIG. 6 is an illustration of an EEG recording 4000 containing a seizure, a muscle artifact and an eye movement artifact. FIG. 7 is an illustration of the EEG recording 5000 of FIG. 6 with the muscle artifact removed. FIG. 8 is an illustration of the EEG recording 6000 of FIG. 7 with the eye movement artifact removed.

An additional description of analyzing EEG recordings is set forth in Wilson et al, U.S. Patent Application Number 13/684469, filed on November 23, 2012, for a *User Interface For Artifact Removal In An EEG.* An additional description of analyzing EEG recordings is set forth in Wilson et al, U.S. Patent Application Number 13/684556, filed on November 25, 2012, for a *MethodAnd System For Detecting And Removing EEG Artifacts.* Once an amount of artifact is detected in an EEG, the processor (processing engine) is configured to generate a graphical display of an amount of artifact present in an EEG recording, which is displayed as shown in FIG. 1.

## Claims

1. A method for displaying an amount of artifact present in an EEG recording, the method comprising:
generating an EEG recording (51) from a machine comprising a plurality of electrodes (35), an amplifier (42) and processor;
analyzing the EEG recording to determine an amount of artifact present in the EEG recording; and
displaying the amount of a plurality of artifact types present in the EEG recording on a display, wherein the amount of the plurality of artifact types present in the EEG recording is displayed as a plurality of horizontal lines, **characterised in that** the plurality of horizontal lines comprises a horizontal line for a muscle artifact, a horizontal line for a chewing artifact, a horizontal line for a vertical eye movement artifact, and a horizontal line for a lateral eye movement artifact.

2. The method according to claim 1 wherein a depth of color of a horizontal line of the plurality of horizontal lines indicates the amount of a specific artifact detected in the EEG recording over a given time period.

3. A system (20) for displaying an amount of artifact present in an EEG recording (51), the system comprising:
a plurality of electrodes (35) for generating a plurality of EEG signals; a processor connected to the plurality of electrodes to generate an EEG recording from the plurality of EEG signals; and
a display (50) connected to the processor for displaying the EEG recording;
wherein the processor is configured to graphically display an amount of a plurality of artifact types present in an EEG recording, wherein the amount of the plurality of artifact types present in the EEG recording is displayed as a plurality of horizontal lines, **characterised in that** the plurality of horizontal lines comprises a horizontal line for a muscle artifact, a horizontal line for a chewing artifact, a horizontal line for a vertical eye movement artifact, and a horizontal line for a lateral eye movement artifact.

4. The system according to claim 3 wherein a depth of color of a horizontal line of the plurality of horizontal lines indicates the amount of a specific artifact detected in the EEG recording over a given time period.

## Patentansprüche

1. Ein Verfahren zur Anzeige einer Menge von Artefakten in einer EEG-Aufzeichnung, wobei das Verfahren umfasst:
Erzeugen einer EEG-Aufzeichnung (51) von einer Maschine, die eine Vielzahl von Elektroden (35), einen Verstärker (42) und einen Prozessor umfasst;
Analysieren der EEG-Aufzeichnung, um eine Menge eines Artefakts in der EEG-Aufzeichnung zu bestimmen; und
Anzeigen der Menge einer Vielzahl von Artefaktformen in der EEG-Aufzeichnung auf einer Anzeige, wobei die Menge der Vielzahl von Artefaktformen in der EEG-Aufzeichnung als eine Vielzahl von horizontalen Linien angezeigt wird, **dadurch gekennzeichnet, dass** die Vielzahl von horizontalen Linien umfasst: eine horizontale Linie für ein Muskelartefakt, eine horizontale Linie für ein Kau-Artefakt, eine horizontale Linie für ein vertikales Augenbewegungs-Artefakt und eine horizontale Linie für ein seitliches Augenbewegungs-Artefakt.

2. Das Verfahren nach Anspruch 1, wobei eine Farbtiefe einer horizontalen Linie aus der Vielzahl der horizontalen Linien die Menge eines spezifischen Artefakts anzeigt, das in der EEG-Aufzeichnung über eine gegebene Zeitspanne festgestellt wird.

3. Ein System (20) zur Anzeige einer Menge eines Artefakts in einer EEG-Aufzeichnung (51), wobei das System umfasst:
eine Vielzahl von Elektroden (35) zum Erzeugen einer Vielzahl von EEG-Signalen, einen Prozessor, der mit der Vielzahl von Elektroden verbunden ist, um eine EEG-Aufzeichnung aus der Vielzahl von EEG-Signalen zu erzeugen, und
eine Anzeige (50), die mit dem Prozessor verbunden ist, um die EEG-Aufzeichnung anzuzeigen;
wobei der Prozessor konfiguriert ist, um eine Menge einer Vielzahl von Artefaktformen in einer EEG-Aufzeichnung grafisch anzuzeigen, wobei die Menge der Vielzahl von Artefaktformen in der EEG-Aufzeichnung als eine Vielzahl von horizontalen Linien angezeigt wird, **dadurch gekennzeichnet, dass** die Vielzahl von horizontalen Linien umfasst: eine horizontale Linie für ein Muskelartefakt, eine horizontale Linie für ein Kau-Artefakt, eine horizontale Linie für ein vertikales Augenbewegungs-Artefakt und eine horizontale Linie für ein seitliches Augenbewegungs-Artefakt.

4. Das System nach Anspruch 3, wobei eine Farbtiefe einer horizontalen Linie aus der Vielzahl der horizontalen Linien die Menge eines spezifischen Artefakts anzeigt, das bei der EEG-Aufzeichnung über eine gegebene Zeitspanne festgestellt wird.

## Revendications

1. Procédé d'affichage d'une quantité d'artefact présent dans un enregistrement EEG, le procédé comprenant :
générer un enregistrement EEG (51) à partir d'une machine comprenant une pluralité d'électrodes (35), un amplificateur (42) et un processeur ;
analyser l'enregistrement EEG pour déterminer une quantité d'artefact présent dans l'enregistrement EEG ; et
afficher la quantité d'une pluralité de types d'artefact présents dans l'enregistrement EEG sur un affichage, dans lequel la quantité de la pluralité de types d'artefact présents dans l'enregistrement EEG est affichée sous forme d'une pluralité de lignes horizontales,
**caractérisé en ce que** la pluralité de lignes horizontales comprend une ligne horizontale pour un artefact musculaire, une ligne horizontale pour un artefact de mastication, une ligne horizontale pour un artefact de mouvement oculaire vertical, et une ligne horizontale pour un artefact de mouvement oculaire latéral.

2. Procédé selon la revendication 1, dans lequel une profondeur de couleur d'une ligne horizontale de la pluralité de lignes horizontales indique la quantité d'un artefact spécifique détecté dans l'enregistrement EEG sur une période de temps donnée.

3. Système (20) pour afficher une quantité d'artefact présent dans un enregistrement EEG (51), le système comprenant :
une pluralité d'électrodes (35) pour générer une pluralité de signaux EEG;
un processeur connecté à la pluralité d'électrodes pour générer un enregistrement EEG à partir de la pluralité de signaux EEG ; et
un affichage (50) connecté au processeur pour afficher l'enregistrement EEG ;
dans lequel le processeur est configuré pour afficher graphiquement une quantité d'une pluralité de types d'artefact présents dans un enregistrement EEG, dans lequel la quantité de la pluralité de types d'artefact présents dans l'enregistrement EEG est affichée sous la forme d'une pluralité de lignes horizontales,
**caractérisé en ce que** la pluralité de lignes horizontales comprend une ligne horizontale pour un artefact musculaire, une ligne horizontale pour un artefact de mastication, une ligne horizontale pour un artefact de mouvement oculaire vertical, et une ligne horizontale pour un artefact de mouvement oculaire latéral.

4. Système selon la revendication 3, dans lequel une profondeur de couleur d'une ligne horizontale de la pluralité de lignes horizontales indique la quantité d'un artefact spécifique détecté dans l'enregistrement EEG sur une période de temps donnée.
